# NEW EUROPEAN PATENT SPECIFICATION

(11) **EP 1 011 785 B2**
(45) Date of publication and mention of the opposition decision: **15.07.2015**
(45) Mention of the grant of the patent: 03.01.2007
(21) Application number: 98904865.7
(22) Date of filing: 05.02.1998
(51) Int. Cl.: A61M 5/158

(54) **INJECTOR FOR A SUBCUTANEOUS INFUSION SET**
INJEKTIONSVORRICHTUNG FÜR EIN SUBKUTANES INFUSIONSGERÄT
INJECTEUR POUR DISPOSITIF DE PERFUSION SOUS-CUTANEE

(30) Priority: 05.02.1997 US 795968
(43) Date of publication of application: 28.06.2000
(73) Proprietor: Medtronic MiniMed, Inc., Northridge, CA 91325-1219 (US)
(72) Inventor: FUNDERBURK, Jeffery, V., Granada Hills, CA 91344 (US); MARANO, April, A., Los Angeles, CA 90049 (US); Field Jeffrey, California 93012 (US)
(74) Representative: Ruschke, Hans Edvard
(86) International application number: PCT/US1998/002031
(87) International publication number: WO 1998/033549

(56) References cited:
- US-A- 4 755 173
- US-A- 5 122 119
- US-A- 5 665 071

## Description

### BACKGROUND OF THE INVENTION

This invention relates generally to an injector for automatic placement of a medical needle through the skin of a patient. More specifically, this invention relates to a compact and easily operated injector for placement of an insertion needle and related cannula of a subcutaneous infusion set or the like through the skin of a patient with a controlled force and insertion speed, and particularly wherein the injector is adapted for use by the patient.

Medical needles are widely used in the course of patient care and treatment, particularly with respect to the delivery of selected medications to a patient. In one common form, hollow hypodermic needles are employed for transcutaneous delivery of a selected medication from a syringe or the like. In another common form, insertion needles are employed for transcutaneous placement of a soft and relatively flexible tubular cannula, followed by insertion needle removal and subsequent infusion of medical fluid to the patient through the cannula.

In certain medical treatment regimens, it may be necessary or desirable for the patient to transcutaneously place the medical needle. For example, diabetic patients frequently self-administer insulin injections or periodically place a subcutaneous infusion set for subsequent programmable delivery of insulin by means of a medication infusion pump of the general type described in U.S. Patent 4,685,903. Such subcutaneous infusion sets are disclosed, for example, in U.S. Patents 4,755,173; 5,176,662; and 5,257,980.

Some patients are reluctant or hesitant to pierce their own skin with a medical needle, and thus encounter difficulties in correct needle placement for proper administration of the medication. Such difficulties can be attributable to insufficient manual dexterity or skill to achieve proper needle placement or alternately to anxiety associated with anticipated discomfort as the needle pierces the skin. This problem can be especially significant with medications delivered via a subcutaneous infusion set, since incorrect placement can cause kinking of the cannula and resultant obstruction of medication flow to the patient. Cannula kinking can be due to infusion set placement at an incorrect angle relative to the patient's skin, and/or needle placement with an incorrect force and speed of insertion.

The present invention relates to an automatic injector, particularly for use with a subcutaneous infusion set, for quickly and easily placing an insertion needle and related cannula through the skin of a patient at the correct insertion angle, and with a speed and force of insertion which minimizes patient discomfort.

### SUMMARY OF THE INVENTION

The present invention provides an injector as defined by claim 1.

The injector is designed to place the needle through the skin at a selected insertion angle and with a controlled force and speed of insertion, to ensure proper needle placement with minimal patient discomfort. The injector is particularly designed to meet these objectives, while safeguarding against undesired projection of the medical needle through free space, in the event that the injector is actuated in spaced relation to the patient's skin.

The injector comprises a spring-loaded plunger having a head for receiving and supporting an infusion set in a position with an insertion needle and related cannula projecting outwardly for transcutaneous placement through the skin of a patient. The plunger is designed for retraction and retention within a barrel to a cocked position with a drive spring compressed in a manner applying a predetermined spring force to the plunger head. A front or nose end of the injector barrel is designed for pressed placement against the skin of a patient, at a selected needle insertion site, and in an orientation with the needle disposed at a correct or desired insertion angle. A trigger member is operable to release the plunger and thereby permit the drive spring to carry the insertion set toward the patient's skin with a controlled force and speed, resulting in proper transcutaneous placement of the insertion needle and related cannula with minimal patient discomfort.

The plunger head includes a safety lock mechanism to retain the infusion set against projection from the injector barrel. In one preferred form, the safety lock mechanism comprises at least one and preferably a pair of safety lock arms for engaging and retaining the infusion set when the plunger is retracted from a fully advanced position. Each safety lock arm includes a cam lobe for engaging an appropriately shaped recess on the infusion set to prevent release thereof from the plunger head, unless and until the plunger head is returned to the fully advanced position. In such fully advanced position, the shape of the cam lobe permits quick and easy separation of the injector from the infusion set with a minimal separation force.

In operation, the safety lock arms thus prevent projection of the infusion set from the injector, in the event that the trigger member is actuated with the nose end of the barrel spaced from the skin of a patient. In that event, the plunger head is advanced with the controlled force and speed to the fully advanced position, but the infusion set is not thrown from the injector as a projectile. Instead, the infusion set travels rapidly with the plunger head to the fully advanced position, whereat the injector can be separated with minimal separation force from the infusion set.

In an alternative preferred form, the safety lock mechanism comprises a plunger head having a cylindrical shape defining a forwardly open cavity for receiving and supporting an infusion set with the insertion needle and cannula projecting outwardly. In this embodiment, the plunger head includes a radially inwardly projecting rim at a forward or nose end thereof, wherein the rim defines an oval-shaped opening. The size of the rim opening permits relatively free reception of a hub on the infusion set, with the infusion set oriented at an angle relative to a central axis of the plunger head and barrel. The infusion set is then reoriented to align the insertion needle coaxially with the central axis of the barrel and plunger head, so that the rim is received into a recess on the infusion set and functions to retain the infusion set against undesired release from the injector during spring-driven placement of the needle. After needle placement, the injector is released from the infusion set with minimal separation force by orienting the injector angularly relative to the infusion set to permit free slide out passage of the hub through the oval rim opening.

Other features and advantages of the present invention will become more apparent from the following detailed description which illustrates, by way of example, the principle of the invention.

### DETAILED DESCRIPTION OF THE PREFERRED EMBODIMENTS

An injector referred to is provided for quick and easy transcutaneous placement of a medical needle, particularly such as an insertion needle 12 of the type provided with a subcutaneous infusion set. The injector includes a trigger-type actuator mechanism for transcutaneous placement of the insertion needle with a controlled speed and force, and with the insertion needle oriented at a desired angular position relative to the skin of the patient.

The automatic injector of the present invention is particularly designed for placement of the insertion needle of a subcutaneous infusion set of the general type shown and described in U.S. Patents 4,755,173; 5,176,662; and 5,257,980. Such infusion sets are used to infuse medical fluids such as selected medications to a patient, with one example being the administration of insulin to a diabetic by operation of a programmable medication infusion pump of the type described in U.S. Patent 4,685,903. The insertion needle is connected to a hub at a rear or proximal end thereof, and protrudes through a housing of the infusion set wherein the housing defines an internal chamber (not shown) for receiving medication via infusion tubing. An enlarged base, typically in the form of resilient or flexible wings is provided on the housing for stable affixation to the skin of a patient. The insertion needle protrudes downwardly through the housing and the winged base to extend through a soft and flexible cannula. The insertion needle is provided for transcutaneous placement of the cannula after which the insertion needle is retracted from the set to permit medication delivery through the cannula to the patient.

The injector of a first embodiment of the present invention represents a simple device which can be used by the patient to quickly and easily place the subcutaneous infusion set in a proper transcutaneous position and orientation, at a selected medication insertion site. The injector is designed to project the infusion set toward the patient's skin at a controlled force and speed for quickly piercing the skin in a manner insuring proper placement of the insertion needle and cannula, while minimizing patient anxiety and/or discomfort. Improper and/or partial placement of the insertion needle is thus avoided.

In general terms, as shown in one preferred form the injector comprises a cylindrical forward barrel having a plunger mounted therein for longitudinal sliding movement within a hollow bore between a forward advanced position and a rearward retracted position. The plunger has a head at a forward end thereof for releasibly receiving and retaining the subcutaneous infusion set in a manner to be described in more detail. A rear end of the plunger cooperates with a trigger-type actuator assembly mounted on the rear end of the barrel. The trigger actuator assembly is adapted to hold the plunger in a retracted position, against the force of a compressed drive spring. A trigger button of the actuator assembly is adapted for fingertip depression to release the plunger for spring-loaded travel toward the advanced position, and corresponding transcutaneous placement of the insertion needle through the patient's skin.

Considering construction of details of the injector barrel, the forward or nose end defines a flat and generally planar surface for placement against the skin of a patient with a longitudinal axis of the barrel oriented generally perpendicular to the patient's skin. The barrel has a size and shape for substantially mated sliding fit reception of the infusion set with the insertion needle and related cannula projecting in a direction for placement on a patient. In this regard, the nose end of the barrel defines an opposed pair of relatively wide and open-ended cut outs for slide-fit reception of the oppositely projecting base wings. A narrower slot is also formed in the barrel nose end, at a location for slide-fit reception of the infusion tubing attached to the infusion set. Thus, the forward or nose end of the barrel accommodates sliding reception of the subcutaneous infusion set therein for movement along the cut outs and the slot between the advanced position disposed substantially at the forwardmost end of the barrel, and the retracted position with the base wings and infusion tubing positioned substantially at the inboard ends of the cut outs and the associated slot.

The plunger includes the head of generally cylindrical shape for slide-fit reception within the injector barrel. A forward end of the head includes a cylindrical counterbore recess for receiving the hub and housing of the infusion set, with the enlarged base wings fitted against a pair of outwardly protruding backstop flanges adapted for slide-fit reception within the cut outs in the barrel nose end. A pair of track arms protrude rearwardly from the plunger head and include out-turned latch fingers for guided reception within longitudinally extending track slots formed in the barrel at a location spaced aft from the barrel nose end. These track arms thus cooperate with the barrel track slots to guide the plunger between the advanced position and the retracted position.

The plunger also includes a central drive stem which protrudes rearwardly from the plunger head within the barrel interior. The rearward end of the drive stem is longitudinally split to define a pair of trigger arms which have out-turned trigger fingers on the rearward ends thereof.

The trigger-type actuator assembly is mounted on the rearward end of the injector barrel and generally functions to releasibly retain the plunger in a retracted and cocked position, ready for rapid and spring-loaded actuation upon depression of the trigger button to place the infusion set on the patient. More particularly, the trigger assembly comprises a main support cap having a mounting sleeve protruding in a press-fit and preferably adhesively connected manner into the rear or aft end of the injector barrel. The drive spring comprises a coil spring positioned about the drive stem on the plunger and reacts between a rearward face of the plunger head and a shoulder on the support cap. The drive spring normally biases the plunger toward the advanced position. However, an infusion set seated in the plunger head can be pressed rearwardly against the plunger to move the plunger tto the retracted position with the trigger fingers passed through a conical or tapered latch bore formed in the support cap to engaging a shoulder on an opposite side of the support cap. In this regard, the trigger fingers have ramped outboard faces to accommodate movement of the fingers radially toward each other as they pass through the latch bore. When the trigger fingers pass entirely through the bore the spring resilience of the trigger arms is sufficient to spread the trigger fingers so that they engage the shoulder. In this retracted plunger position, the drive spring is retained in a compressed and cocked condition, with the infusion set including the insertion needle and related cannula withdrawn into the interior of the barrel in spaced relation to the patient's skin.

The trigger actuator assembly additionally includes an actuator pin mounted within a noncircular bore formed in the support cap for longitudinal sliding movement through a short stroke, relative to the plunger. In this regard, the actuator pin includes one or more noncircular lands for slide-fit reception within the bore to prevent actuator pin rotation therein. The actuator pin is held within the bore by a stepped lock ring which is retained against a rearward end of the support cap by a press-fit outer retainer sleeve having an intumed rim at the rearward end thereof. Importantly an oblong land is formed on the actuator pin for mated slide-fit reception through an oblong recess formed in the lock ring. A return spring is carried within the support cap bore and reacts between the shoulder and a nose end of the actuator pin for biasing the actuator pin rearwardly within the support cap.

The rearmost end of the actuator pin defines the trigger button. The trigger button can be depressed with a fingertip to move the actuator pin through a short stroke against the return spring in a direction toward the trigger fingers at the rear end of the plunger. The actuator pin has a hollowed cylindrical forward tip with a diametric size for engaging and squeezing the trigger fingers together at the rear end of the plunger, in a manner enabling those trigger fingers to pass back through the tapered conical latch bore. As soon as the trigger fingers thus release from engagement with the shoulder on the support cap the drive spring translates the plunger with the infusion set thereon with a rapid and controlled force and speed toward the advanced position, resulting in transcutaneous placement of the needle and cannula. Importantly, the spring rate characteristics of the drive spring and the distance of plunger stroke are chosen for a substantially optimized and proper transcutaneous placement of the needle and cannula all in a manner which minimizes patient discomfort during the needle placement procedure. Moreover, by forming the nose end of the injector barrel with a squared-off shape as shown, the injector can be easily oriented substantially perpendicular to the skin for proper placement of the infusion set.

Depression of the actuator pin by means of the trigger button requires the lock ring to be rotatably oriented in a position aligning the oblong recess therein with the oblong land on the actuator pin. Accordingly, when these oblong structures are rotationally aligned, the injector is armed for trigger button depression and corresponding release of the retracted and cocked plunger. However, the lock ring can be rotated relative to the actuator pin to misalign these oblong structures, whereupon the actuator pin is locked in a rearward position against depression and actuation. A set pin on the lock ring may be provided and received within an arcuate notch formed in the retainer sleeve flange rim to permit lock ring rotation back-and-forth through a part circle increment, on the order of about degrees. Appropriate indicia may be applied to the retainer sleeve rim such as the letter "L" for "locked" and the letter "A" for "armed" to provide a visual indication of the setting of the trigger assembly.

In accordance with one primary aspect of the invention, the plunger head additionally includes a safety lock mechanism in the form of a pair of safety lock arms pivotally carried in narrow slots formed in the plunger head. These safety lock arms have rearward ends connected to the head by pivot pins and forward ends defining contoured lock fingers which protrude into the plunger head recess. The safety lock arms and their associated lock fingers have a size and shape so that the fingers can engage and retain the hub of the infusion set for example, by fitting into a recess defined between the hub and housing of the infusion set. Importantly, the locations of the lock arm pivot points are chosen to insure that the lock arms engage and retain the infusion set whenever the plunger is moved from the advanced position toward and to the retracted position. When the plunger reaches the fully advanced position, the safety lock arms including their respective pivot pins are disposed within the wide cut outs and are therefore free to swing outwardly, relative to the insertion set to accommodate separation of the insertion set from the injector with a substantially minimum separation force. This configuration has been found to be highly effective as a safeguard to prevent the infusion set from being thrown as a projectile from the injector in the event that the trigger assembly is activated without prior placement of the injector firmly against the patient's skin.

In use, the subcutaneous infusion set can be placed quickly and easily into the open nose end of the injector barrel within the recess formed in the plunger head. Such assembly of the insertion set with the injector requires simple alignment of the base wings and infusion tubing with the appropriate cut outs and slots formed in the nose end of the barrel. The infusion set and plunger can then be manually retracted rearwardly, against the drive spring to the retracted position with the plunger cocked and latched. The injector can then be placed firmly against the patient's skin with the infusion set supported in the proper orientation and at a predetermined distance from the skin. Simple depression of the trigger button releases the cocked plunger for spring-loaded travel rapidly albeit with a controlled speed and force of insertion, to ensure penetration of the patient's skin with minimal discomfort, and in a manner which properly places the insertion needle and cannula. The safety lock arms prevent accidental projection of the infusion set through free space, in the event that the trigger button is prematurely depressed. When the infusion set is properly placed, however, the safety lock arms release from the infusion set with minimal force, for easy separation of the injector from the insertion set.

Following separation of the injector from the placed infusion set the insertion needle can be withdrawn quickly and easily from the cannula. Thereafter, the infusion set can be used in a normal manner to deliver a selected medication through the infusion tubing and cannula to the patient.

In a second embodiment a modified injector a constructed from a reduced number of parts and including an alternative safety lock mechanism for preventing undesired projection of the infusion set through free space in the event of injector operation without placing the nose end thereof firmly against the skin of a patient. However, the alternative safety lock mechanism again permits quick and easy separation of the injector from the infusion set with minimal separation force.

In general, the modified injector comprises a plunger and a trigger-type actuator assembled with a generally cylindrical hollow barrel. The plunger has a generally cylindrical plunger head which defines a counterbore recess for receiving and retaining the hub of the infusion set. A radially inwardly projecting rim is formed on the plunger head generally at a leading or nose end of the recess wherein this rim has a noncircular and preferably oval or elliptical shape to accommodate reception of the hub into the recess provided that the hub is oriented angularly relative to a central longitudinal axis of the plunger and barrel. Similar angular orientation of these components accommodates quick and easy separation thereof. However, when the infusion set is oriented with the medical needle aligned coaxially with the barrel center axis, a portion of the rim projects into the insertion set recess to prevent release of the insertion set from the injector.

More specifically the barrel again has a forward or nose end defining a flat and generally planar surface for firm placement against the skin of a patient. The nose end of the barrel has a pair of relatively wide and generally opposed cut outs formed therein for slide-fit reception of the base wings of the infusion set in combination with a narrower slot for slide-fit reception of the infusion tubing.

The plunger is slidably fitted into the barrel for movement between an advanced position and a retracted position. The plunger includes the modified plunger head of generally cylindrical shape, formed preferably to include a shallow notch or groove in one side thereof for slide-fit reception of the infusion tubing on the infusion set. In this regard, the plunger head groove is formed in a position aligned with the narrow slot in the nose end of the barrel.

The plunger head is formed integrally with a drive stem which projects rearwardly within the barrel interior. The drive stem is flanked by and formed integrally with a pair of rearwardly projecting track arms which have latch fingers formed at the rear ends thereof. These latch fingers are received slidably within longitudinally extending track slots formed in the barrel and function to guide the plunger between the advanced and retracted positions.

The plunger additionally includes a pair of trigger arms which project generally rearwardly from a rear end of the drive stem and have out-turned trigger fingers at the rear ends thereof. These trigger fingers are adapted and sized for partial radial compression toward each other as they ride within the barrel base when the plunger is displaced from the advanced position to the retracted position. As the retracted position is reached, the trigger fingers are spring-loaded by the resiliency of the trigger arms to move outwardly for partial reception into relatively short trigger slots formed in the barrel. In this position the triggers fingers retain the plunger in the retracted position.

A drive spring is mounted within the barrel to react between the trigger-type actuator and the plunger in the same manner as previously described with respect to the first embodiment. In this regard, the trigger actuator comprises a generally cylindrical actuator sleeve mounted slidably within the barrel at the rear or upper end thereof. This actuator sleeve has a tapered or ramped leading edge face for engaging matingly shaped ramped outer faces of the trigger fingers to radially compress the trigger arms and release the plunger for spring-loaded travel from the retracted and cocked position to the advanced position. A trigger button is formed integrally with the actuator sleeve and is exposed for fingertip depression at the rear or top of the barrel to move the actuator sleeve into releasing engagement with the trigger fingers.

The triggers button extends through an opening formed in the rear of the barrel generally within a lock sleeve formed integrally with the barrel. The lock sleeve defines an oppositely formed pair of guide slots for aligned reception of a pair of outwardly radiating lock tabs formed on the trigger button. When the tabs and rotationally aligned with the guide slots the trigger button can be depressed to actuate the spring-locked plunger, as described. However, the lock tabs have sufficient length to permit fingertip rotation of the actuator to reposition the tabs within shallow lock grooves formed adjacent the guide slots. When the tabs are seated in the lock grooves the lock sleeve blocks depression of the triggers button and thereby locks the injector against actuation. Return rotation of the actuation to re-align the tabs with the guide slots is required before the injector can be activated.

In accordance with one primary aspect of the invention, the plunger head includes the safety lock mechanism in the form of the noncircular rim at the leading end of the recess in the plunger head. The rim has a generally elliptical shape defining a major axis that is greater than the diameter of the hub on the infusion set and a minor axis that is less than the hub diameter. With this geometry, and by providing sufficient axial depth to the plunger head recess the hub can be fitted into the plunger head by angularly orienting the components to permit slide-fit of the hub through the major axis portion of the rim. Subsequent re-orientation of the components to align the medical needle generally coaxially with plunger head enables the minor axis portion of the rim to project into the infusion set recess thereby locking the components together. Thereafter, when the infusion set is placed on the patient the components are easily separated by lifting the injector off the infusion set at the same angle to allow the hub to press freely through the major axis center of the rim. importantly, such engagement and disengagement of the components occurs with essentially no resistance force to separation. The infusion set can be oriented angularly relative to the plunger only when the plunger is in the advanced position, with the adjacent barrel precluding such angular orientation when the plunger is moved rearwardly from the restricted position.

A variety of further modifications and improvements to the automatic injector unit of the present invention will be apparent to persons skilled in the art. Accordingly, no limitation on the invention is intended by way of the foregoing description, except as set forth in the appended claims.

## Claims

1. An injector for transcutaneously placing an insertion needle of a subcutaneous insertion set through the skin of a patient, comprising:
barrel means defining an elongated bore;
plunger means slidably received within said bore for movement between an advanced position and a retracted position, said plunger means including means for receiving and supporting the insertion set in a position with the insertion needle oriented for transcutaneous placement upon movement of said plunger means from said retracted position to said advanced position; and
drive means for urging said plunger means with a controlled force and speed from said retracted position toward said advanced position to transcutaneously place the insertion needle,
wherein the insertion needle is provided as part of an insertion set, said plunger means comprises a plunger head having a recess formed therein for mated slide-fit reception of at least a portion of the insertion set, **characterized in that**
said plunger head further includes safety retainer means for retaining the insertion set on said plunger head during movement from said retracted position to said advanced position, said retainer means permitting separation of the insertion set from said plunger head when said plunger head is in the advanced position.

2. The injector of claim 1 wherein said barrel means has a forward end defining a generally planar surface for placement against the skin of a patient with said barrel means in a predetermined orientation relative to the patient's skin.

3. The injector of claim 1 further including trigger means for actuating said drive means.

4. The injector of claim 3 wherein said trigger means includes a trigger for fingertip depression to actuate said drive means for movement of said plunger means from said retracted position to said advanced position.

5. The injector of claim 3 wherein said drive means comprises spring means for spring-loaded movement of said plunger means from said retracted position to said advanced position.

6. The injector of claim 1 wherein said barrel means and said plunger means include cooperatively engageable track means for guiding movement of said plunger means between said advanced and retracted positions while retaining said plunger means against rotation relative to said barrel means.

7. The injector of claim 1 wherein said plunger head recess comprises a laterally open undercut recess.

8. The injector of claim 2 wherein said plunger means and the insertion needle supported thereby are substantially retracted within said bore when said plunger means is in the retracted position, said barrel means having at least one slot formed therein and extending longitudinally from the forward end thereof.

9. The injector of claim 2 wherein the insertion set comprises at least one outwardly radiation base wing for securing the insertion set to the patient's skin, and at least one outwardly radiating coupling element, said barrel means having at least one cut out and at least one slot formed therein to extend longitudinally from the forward end thereof for respective slide-fit reception of the base wing and coupling element.

10. The injector of claim 1 wherein said retainer means comprising at least one safety lock arm having one end pivotally mounted on said plunger head and an opposite end defining a lock finger for releasibly engaging the insertion needle, said barrel means retaining said safety lock arm against outward pivoting motion relative to said plunger head recess when said plunger means is moved from the advanced position, said safety lock arm being in a position relative to said barrel means for permitting outward pivoting movement with respect to said plunger head recess when said plunger means is in the advanced position.

11. The injector of claim 10 wherein said at least one safety lock arm comprises a pair of safety lock arms each having one end pivotally mounted on said plunger means and an opposite end defining a lock finger for releasibly engaging the insertion needle.

12. The injector of claim 11 wherein said barrel means has a forward end with a pair of open-ended cut outs formed therein, said safety lock arms being disposed generally within said cut outs when said plunger means is in the advanced position.

13. The injector of claim 1 wherein said retainer means comprises a radially inwardly projecting rim formed generally at a leading end of said plunger head, said rim engaging the insertion needle when the needle is generally aligned coaxially within said plunger head and permitting release of the needle when the needle is angularly oriented relative to the plunger head.

14. The injector of claim 13 wherein said shape has a generally elliptical shape.

15. The injector of claim 3 wherein said trigger means includes lock means for releasibly locking said plunger means in the retracted position.

## Patentansprüche

1. Injektionsvorrichtung zum transkutarren Platzieren einer Einführungsnadel eines subkutanen Einfuhrungsbestecks durch die Haut eines Patienten, wobei die Vorrichtung Folgendes umfasst:
Zylindermittel, die eine längliche Bohrung definieren, Druckkolbenmittel, die für eine Bewegung zwischen einer vorgeschobenen. Position und einer eingezogenen Position verschiebbar innerhalb der Bohrung aufgenommen werden, wobei die Druckkolbenmittel Mittel einschließen, um auf eine Bewegung der Druckkolbenmittel von der eingezogenen Position zu der vorgeschobenen Position das Einführungsbesteck aufzunehmen und zu tragen, in einer Position, wobei die Einfuhrungsnadel für ein transkutanes Platzieren ausgerichtet ist, und
Antriebsmittel zum Drücken der Druckkolbenmittel mit einer kontrollierten Kraft und Geschwindigkeit von der eingezogenen Position zu der vorgeschobenen Position hin, um die Einfübrungsnadel transkutan zu platzieren,
wobei die Einführungsnadel als Teil eines Einführungsbestecks bereitgestellt wird, wobei die Druckkolbenmittel einen Druckkolbenkopf umfassen, der eine in demselben geformte Aussparung für eine passende Gleitsitzaufnahme wenigstens eines Abschnitts des Einführungsbestecks hat, **dadurch gekennzeichnet, dass**
der Druckkolbenkopf ferner Sicherheitsfesthaltemittel einschließt, um das Einführungsbesteck während der Bewegung von der eingezogenen Position zu der vorgeschobenen Position am Druckkolbenkopf festzuhalten, wobei die Sicherheitsfesthaltemittel ein Trennen des Einführungsbestecks vom Druckkolbenkopf erlauben, wenn der Druckkolbenkopf in der vorgeschobenen Position ist.

2. Injektionsvorrichtung nach Anspruch 1, wobei die Zylindermittel ein vorderes Ende haben, das eine allgemein ebene Fläche zum Platzieren an der Haut eines Patienten definiert, wobei sich die Zylindermittel in einer vorher festgelegten Ausrichtung im Verhältnis zur Haut des Patienten befinden.

3. Injektionsvorrichtung nach Anspruch 1, die ferner Auslösermittel zum Betätigen der Antriebsmittel einschließt.

4. Injektionsvorrichtung nach Anspruch 3, wobei die Auslösermittel einen Auslöser zum Niederdrücken mit der Fingerspitze einschließen, um das Antriebsmittel für eine Bewegung der Druckkolbenmittel von der eingezogenen Position zu der vorgeschobenen Position zu betätigen.

5. Injektionsvorrichtung nach Anspruch 3, wobei die Antriebsmittel Federmittel für eine gefederte Bewegung der Druckkolbenmittel von der eingezogenen Position zu der vorgeschobenen Position umfassen.

6. Injektionsvorrichtung nach Anspruch 1, wobei die Zylindermittel und die Druckkolbenmittel zusammenwirkend ineinandergreifende Spurmittel einschließen, um die Bewegung der Druckkolbenmittel zwischen der vorgeschobenen Position und der eingezogenen Position zu führen, während die Druckkolbenmittel gegen eine Drehung im Verhältnis zu den Zylindermitteln festgehalten werden.

7. Injektionsvorrichtung nach Anspruch 1, wobei die Druckkolbenkopf-Aussparung eine seitlich offene unterschnittene Aussparung umfasst.

8. Injektionsvorrichtung nach Anspruch 2, wobei die Druckkolbenmittel und die durch dieselben getragene Einführungsnadel wesentlich innerhalb der Bohrung eingezogen sind, wenn sich die Druckkolbenmittel in der eingezogenen Position befinden, wobei die Zylindermittel wenigstens einen Schlitz haben, der in denselben geformt ist und sich in Längsrichtung vom vorderen Ende derselben aus erstreckt.

9. Injektionsvorrichtung nach Anspruch 2, wobei das Einführungsbesteck wenigstens einen nach außen ausstrahlenden Basisflügel zum Befestigen des Einführungsbestecks an der Haut des Patienten und wenigstens ein nach außen ausstrahlendes Kopplungselement umfasst, wobei die Zylindermittel für eine jeweilige Gleitsitzaufnahme des Basisflügels und des Kopplungselements wenigstens einen Ausschnitt und wenigstens einen Schlitz haben, die so in denselben geformt sind, dass sie sich in Längsrichtung vom vorderen Ende derselben aus erstrecken.

10. Injektionsvorrichtung nach Anspruch 1, wobei die Festhaltemittel wenigstens einen Sicherheitsverriegelungsarm umfassen, der ein schwenkbar am Druckkolbenkopf angebrachtes Ende und ein entgegengesetztes Ende, das einen Verriegelungsfinger zum lösbaren Ineingriffnehmen der Einfübrungsnadel definiert, hat, wobei die Zylindermittel den Sicherheitsverriegelungsarm gegen eine Auswärts-Schwenkbewegung im Verhältnis zur Druckkolbenkopf-Aussparung festhält, wenn die Druckkolbenmittel aus der vorgeschobenen Position bewegt sind, wobei sich der Sicherheitsverriegelungsarm im Verhältnis zu den Zylindermitteln in einer Position befindet, um eine Auswärts-Schwenkbewegung im Verhältnis zur Druckkolbenkopf-Aussparung zu erlauben, wenn sich die Druckkolbenmittel in der vorgeschobenen Position befinden.

11. Injektionsvorrichtung nach Anspruch 10, wobei der wenigstens eine Sicherheitsverriegelungsarm ein Paar von Sicherheitsverriegelungsarmen umfasst, die jeder ein schwenkbar am Druckkolbenkopf angebrachtes Ende und ein entgegengesetztes Ende, das einen Verriegelungsfinger zum lösbaren Ineingriffnehmen der Einführungsnadel definiert, haben.

12. Injektionsvorrichtung nach Anspruch 11, wobei die Zylindermittel ein vorderes Ende mit einem Paar von in denselben geformten offenendigen Ausschnitten haben, wobei die Sicherheitsverriegelungsarme allgemein innerhalb der Ausschnitte angeordnet sind, wenn sich die Druckkolbenmittel in der vorgeschobenen Position befinden.

13. Injektionsvorrichtung nach Anspruch 1, wobei die Festhaltemittel einen in Radialrichtung nach innen vorspringenden Rand umfassen, der allgemein an einem vorderen Ende des Druckkolbenkopfes geformt ist, wobei der Rand die Einfühntigsnadel im Eingriff nimmt, wenn die Nadel allgemein koaxial innehalb des Druckkolbenkopfes ausgerichtet ist, und ein Lösen der Nadel erlaubt, wenn die Nadel im Verhältnis zum Druckkolbenkopf winklig ausgerichtet ist.

14. Injektionsvorrichtung nach Anspruch 13, wobei die Form eine allgemein elliptische Form hat.

15. Injektionsvorrichtung nach Anspruch 3, wobei die Auslösermittel Verriegelungsmittel zum lösbaren Verriegeln der Druckkolbenmittel in der eingezogenen Position einschließen.

## Revendications

1. Injecteur pour placer en transcutanée une aiguille d'insertion d'un set d'insertion sous-cutané à travers la peau d'un patient, comprenant:
un moyen de cylindre, définissant un alésage allongé;
un moyen de piston reçu par glissement dans ledit alésage en vue d'un déplacement entre une position avancée et une position rétractée, ledit moyen de piston englobant un moyen pour recevoir et supporter le set d'insertion dans une position dans laquelle l'aiguille d'insertion est orientée en vue d'un placement transcutané lors du déplacement dudit moyen de piston de ladite position rétractée vers ladite position avancée; et
un moyen d'entraînement pour pousser ledit moyen de piston par une force et une vitesse contrôlées de ladite position rétractée vers ladite position avancée en vue de placement transcutané de ladite aiguille d'insertion;
l'aiguille d'insertion faisant partie d'un set d'insertion, ledit moyen de piston comprenant une tête de piston comportant un évidement qui y est formé en vue d'une réception par ajustement par glissement complémentaire d'au moins une partie du set d'insertion; **caractérisé en ce que**
ladite tête de piston englobe en outre un moyen de retenue de sécurité pour retenir le set d'insertion sur ladite tête du piston au cours du déplacement de ladite position rétractée vers ladite position avancée, ledit moyen de retenue permettant la séparation du set d'insertion de ladite tête de piston lorsque ladite tête de piston se trouve dans la position avancée.

2. Injecteur selon la revendication 1, dans lequel ledit moyen de cylindre comporte une extrémité avant, définissant une surface généralement plane destinée à être placée contre la peau d'un patient, ledit moyen de cylindre se trouvant dans une orientation prédéterminée par rapport à la peau du patient.

3. Injecteur selon la revendication 1, englobant en outre un moyen de déclenchement pour actionner ledit moyen d'entraînement.

4. Injecteur selon la revendication 3, dans lequel ledit moyen de déclenchement englobe un élément de déclenchement destiné à être enfoncé par la pointe d'un doigt pour actionner ledit moyen d'entraînement en vue de déplacer ledit moyen de piston de ladite position rétractée vers ladite position avancée.

5. Injecteur selon la revendication 3, dans lequel ledit moyen d'entraînement comprend un moyen de ressort en vue d'un déplacement chargé par ressort dudit moyen de piston de ladite position rétractée vers ladite position avancée.

6. Injecteur selon la revendication 1, dans lequel ledit moyen de cylindre et ledit moyen de piston englobent des moyens de piste à engagement coopératif pour guider le déplacement dudit moyen de piston entre lesdites positions avancée et rétractée tout en empêchant une rotation dudit moyen de piston par rapport audit moyen de cylindre.

7. Injecteur selon la revendication 1, dans lequel ledit évidement de ladite tête de piston comprend un évidement dégagé latéralement ouvert

8. Injecteur selon la revendication 2, dans lequel ledit moyen de piston et l'aiguille d'insertion supportée par celui-ci sont pratiquement rétractés dans ledit alésage lorsque ledit moyen de piston se trouve dans la position rétractée, ledit moyen de cylindre comportant au moins une fente qui y est formée et s'étendant longitudinalement à partir de l'extrémité avant correspondante.

9. Injecteur selon la revendication 2, dans lequel le set d'insertion comprend au moins une aile de base rayonnant vers l'extérieur pour fixer le set d'insertion sur la peau du patient, et au moins un élément d'accouplement rayonnant vers l'extérieur ledit moyen de cylindre comportant au moins une entaille et au moins une fente qui y sont formée, s'étendant longitudinalement à partir de l'extrémité avant correspondante en vue d'une réception par ajustement par glissement de l'aile de base et de l'élément d'accouplement.

10. Injecteur selon la revendication 1, dans lequel ledit moyen de retenue comprend au moins un bras de verrouillage de sécurité comportant une extrémité montée par pivotement sur ladite tête de piston, et une extrémité opposée définissant un doigt de verrouillage destiné à s'engager de manière amovible dans l'aiguille d'insertion, ledit moyen de cylindre retenant ledit bras de verrouillage de sécurité contre un déplacement pivotant vers l'extérieur par rapport audit évidement de la tête du piston lorsque ledit moyen de piston est déplacé à partir de la position avancée, ledit bras de verrouillage de sécurité se trouvant dans une position par rapport audit moyen de cylindre destinée à permettre le déplacement pivotant vers l'extérieur par rapport audit évidement de la tête du piston lorsque ledit moyen de piston se trouve dans la position avancée.

11. Injecteur selon la revendication 10, dans lequel ledit au moins un bras de verrouillage de sécurité comprend une paire de bras de verrouillage de sécurité comportant chacun une extrémité montée par pivotement sur ledit moyen de piston et une extrémité opposée définissant un doigt de verrouillage destiné à s'engager de manière amovible dans l'aiguille d'insertion.

12. Injecteur selon la revendication 11, dans lequel ledit moyen de cylindre comporte une extrémité avant avec une paire d'entailles à extrémités ouvertes qui sont formées dans celle-ci, lesdits bras de verrouillage de sécurité étant agencés en général dans lesdites entailles lorsque ledit moyen de piston se trouve dans la position avancée.

13. Injecteur selon la revendication 1, dans lequel ledit moyen de retenue comprend un rebord débordant radialement vers l'intérieur formé en général au niveau d'une extrémité avant de ladite tête de piston, ledit rebord s'engageant dans l'aiguille d'insertion lorsque l'aiguille est en général alignée coaxialement dans ladite tête de piston, et permettant le dégagement de l'aiguille lorsque l'aiguille est orientée à un angle par rapport à la tête de piston.

14. Injecteur selon la revendication 13, dans lequel ladite forme est une forme généralement elliptique.

15. Injecteur selon la revendication 3, dans lequel ledit moyen de déclenchement englobe un moyen de verrouillage pour verrouiller de manière amovible ledit moyen de piston dans la position rétractée.
